# FASCICULE DE BREVET EUROPEEN

(11) **EP 1 017 355 B1**
(45) Date de publication et mention de la délivrance du brevet: **12.12.2001**
(21) Numéro de dépôt: 98945368.3
(22) Date de dépôt: 23.09.1998
(51) Int. Cl.: A61K 6/10

(54) **MATERIAU A BASE DE SILICONE, NOTAMMENT POUR EMPREINTE DENTAIRE ET PROCEDE DE FABRICATION D'UN TEL MATERIAU**
SILIKONENABFORMMASSEN FÜR ZAHNÄRTZLICHE ZWECKE UND VERFAHREN ZU DEREN HERSTELLUNG
MATERIAL BASED ON SILICON, IN PARTICULAR FOR DENTAL IMPRESSION AND METHOD FOR MAKING SUCH MATERIAL

(30) Priorité: 23.09.1997 FR 9712044
(43) Date de publication de la demande: 12.07.2000
(73) Titulaire: Madrigal Finances, 38470 Vinay (FR); Bourgeois, Daniel, 69230 St Genis Laval (FR)
(72) Inventeur: BOURGEOIS, Daniel, F-69230 Saint Genis Laval (FR); CHAUVET, Patrick, F-38210 Saint Quentin sur Isère (FR)
(74) Mandataire: Vuillermoz, Bruno
(86) Numéro de dépôt international: FR9802044
(87) Numéro de publication internationale: WO9915132

(56) Documents cités:
- EP-A- 0 300 309
- EP-A- 0 361 301
- DE-A- 3 423 567
- FR-A- 2 677 013

## Description

L'invention concerne un nouveau matériau à base de silicone. Elle s'applique plus particulièrement mais de façon non limitative, à un nouveau matériau pour empreinte dentaire.

Elle se rapporte également au procédé de fabrication de ce matériau.

De nombreux matériaux sont aujourd'hui utilisés pour la fabrication d'empreintes dentaires et plus particulièrement les matériaux à base de silicone ou d'alginate.

L'invention se rapporte spécifiquement aux matériau à base de silicone. De façon connue, les silicones sont obtenus par réticulation d'une « base » du type organochlorosilane, la réticulation étant initialisée par un catalyseur. Les silicones obtenus sont soit des silicones polycondensés, soit des silicones resultant d'une réaction de polyaddition.

En pratique, les silicones se présentent sous forme d'une pâte épaisse ou fluide, laquelle est déposée sur le fond d'un porte empreinte métallique dont la forme épouse celle de l'arcade dentaire.

L'ensemble porte empreinte/matériau est ensuite mis en bouche et appliqué manuellement sur l'arcade dentaire pendant une durée comprise entre une et quatre minutes, selon les caractéristiques structurelles du matériau utilisé.

Le porte empreinte et le matériau sont ensuite retirés de la bouche puis transmis au prothésiste, lequel fabriquera la prothèse finale.

Même si la durée pendant laquelle le matériau à empreinte reste en bouche est relativement faible, elle est néanmoins suffisante pour que ledit matériau soit recouvert par la salive et le sang présents dans la bouche du patient. Un certain nombre de bactéries, de germes ou autres sont alors susceptibles de se développer non seulement à la surface, mais également dans l'empreinte, ce qui nécessite de désinfecter le matériau dès qu'il est retiré de la bouche du patient, et ce, afin de respecter les règles d'hygiène élémentaires non seulement vis à vis du dentiste mais également vis à vis du prothésiste. En outre, le patient est également concerné dans la mesure où il apparaît que souvent la prothèse voyage entre le prothésiste et le dentiste dans la même boite que celle dans laquelle se trouvait le porte empreinte initial.

Comme procédé de désinfection, on a proposé dans la document US-A-5 624 636 de tremper l'empreinte dentaire dans un bain d'hypochlorite, pendant une durée comprise entre 1 et 5 minutes. On observe une efficacité très relative de ce type de traitement dans la mesure où on n'obtient qu'un traitement de surface.

Pour désinfecter non seulement la surface mais également la masse de l'empreinte, on a proposé dans le document EP-A-0 361 301 d'incorporer en lieu et place de l'eau nécessaire à la préparation d'une empreinte d'alginate, une solution aqueuse et microbicide contenant des sels d'ammonium quaternaire et un composé de diguanidine, de formules spécifiques.

Même si cet antiseptique est efficace, il ne peut être introduit qu'au sein d'un matériau à empreinte hydrophile, tel que l'alginate, mais n'est en aucun cas compatible avec un matériau hydrophobe tel que du silicone. En outre, on se trouve confronté à des problèmes de stabilité dimensionnels de même que des problèmes de stabilité chimique et physique.

En d'autres termes, aucune des solutions proposées à ce jour ne permet de désinfecter efficacement la surface et l'intérieur d'une empreinte à base de silicone, dans la mesure où le traitement par voie externe reste peu efficace et que le traitement par voie interne est effectué à partir d'agents antiseptiques hydrophiles, qui ne peuvent donc être incorporés dans des matériaux hydrophobes.

Le problème que se propose de résoudre l'invention est donc de fournir un matériau à base de silicone pour empreinte dentaire, susceptible d'être désinfecté efficacement.

Pour ce faire, l'invention propose un matériau à base de silicone pour empreinte dentaire comportant un agent antiseptique hydrophobe incorporé dans la masse du silicone et relargable progressivement jusqu'à la surface de l'empreinte.

En d'autres termes, l'invention consiste à avoir incorporé un agent antiseptique compatible avec le silicone, donc hydrophobe et qui présente la capacité d'être libéré dans le temps en développant une action spécifique, à savoir le relargage ionique progressif, permettant la désinfection à la fois de la masse et de la surface de l'empreinte. L'agent antiseptique agit donc par un processus original d'autodésinfection du silicone.

Selon une première caractéristique de l'invention, l'agent antiseptique comprend un agent chélatant, un agent actif sur les membranes phospholipidiques des bactéries ou virus, un agent actif sur les nucléocapsides des virus ou sur tout site récepteur des bactéries.

Comme agent chélatant, on utilise avantageusement l'acide éthylène diamine tétraacétique.

Par ailleurs, l'agent actif sur les membranes phospholipidiques des bactéries ou virus est un sel d'ammonium quaternaire, notamment le chlorure de benzalkonium.

Concernant l'agent actif sur les nucléocapsides des virus ou sur tout site récepteur des bactéries, on utilise préférentiellement le tosylchloramide sodique ou l'un de ses analogues chlorés.

En d'autres termes, l'agent antiseptique agit dans la masse de l'empreinte en relargant progressivement les ions chlore actifs issus du chlorure de benzalkonium et ceux issus du tosylchloramide sodique jusqu'à la surface de l'empreinte. Ainsi, on obtient grâce à l'action interne puis externe de l'agent antiseptique, une empreinte exempte de tous germes, virus ou bactéries infectieuses notamment grâce à la fixation des atomes de chlore sur les sites récepteurs des bactéries et virus.

Selon une forme préférée de réalisation, l'agent antiseptique comprend en masse :
- entre 8 et 80 % d'acide éthylène diamine tétraacétique ;
- entre 2 et 20 % de chlorure de benzaikonium ;
- entre 1 et 20 % de tosylchloramide sodique ou l'un de ses analogues chlorés.

Pour favoriser l'homogénéisation galénique de la préparation antiseptique, celle-ci comprend un double acide aminé, notamment l'aspartam.

Pour obtenir un effet anti-inflammatoire et cicatrisant complémentaire, l'agent antiseptique comprend de l'allantoine.

En tant qu'édulcorant et adjuvant d'isotonicité, l'agent antiseptique comprend un arôme naturel et du sorbitol.

Pour obtenir un matériau exempt de toutes bactéries, germes, virus et autres, la proportion d'agent antiseptique dans ledit matériau est comprise entre 0,1 et 1 % en masse.

Pour une valeur inférieure à 0,1%, la proportion d'agent antiseptique n'est pas suffisante pour obtenir un effet désinfectant.

De même, une proportion d'agent antiseptique supérieure à 1% n'améliore pas le résultat obtenu.

L'invention concerne également le procédé pour la fabrication d'un matériau à base de silicone pour empreinte dentaire, ledit silicone étant obtenu par réticulation d'une base organochlorosilane, ladite réticulation étant initiée par un catalyseur.

Ce procédé se caractérise en ce qu'on incorpore l'agent antiseptique ci-avant décrit dans la base organochlorosilane, puis en ce qu'on malaxe ladite base organochlorosilane avec le catalyseur afin d'obtenir après réticulation, le matériau final.

Selon une caractéristique essentielle du procédé, l'agent antiseptique comprend en masse :
- de 8 à 80 % d'acide éthylène diamine tétraacétique ;
- de 2 à 10 % de chlorure de benzalkonium ;
- de 1 à 10 % de tosylchloramide sodique ou l'un de ses analogues chlorés.

Selon une forme de réalisation préférée du procédé, avant incorporation de l'agent antiseptique dans la base organochlorosilane :
- on mélange préalablement l'agent antiseptique avec une huile de silicone,
- on agite ensuite le mélange obtenu et on le laisse décanter,
- et enfin, après filtration, on incorpore la dispersion obtenue dans la base organochlorosilane.

L'invention et les avantages qui en découlent ressortiront mieux de l'exemple de réalisation qui suit.

On prépare 280 grammes d'un agent antiseptique comprenant un agent chélatant, un agent actif sur les membranes phospholipidiques des bactéries ou virus, un agent actif sur les nucléocapsides des virus ou sur tout site récepteur des bactéries, dont la masse (en gramme) et la nature sont respectivement :

| | |
|---|---|
| EDTA (acide éthylène diamine tétraacétique) | 80 g |
| chlorure de benzalkonium | 38 g |
| tosylchloramide sodique | 38 g |

L'agent antiseptique comprend en outre, en masse:

| | | |
|---|---|---|
| Agent d'homogénéisation | aspartam | 15 g |
| Arôme naturel | menthe | 8 g |
| Agent anti-inflammatoire | allantoïne | 15 g |
| Adjuvant d'isotonicité | sorbitol | 86 g. |

On incorpore la poudre obtenue dans 1 litre d'huile de silicone.

On agite pendant une heure à une température inférieure à 37°C, de préférence environ 30°C.

On laisse décanter le mélange obtenu pendant 24 heures puis on soumet le produit de décantation à une filtration. On récupère ensuite la poudre obtenue.

0,3% en masse de cette poudre sont ensuite incorporés dans 1000 grammes d'une base organochlorosilane.

On fabrique ensuite le silicone final, comme il est connu de le faire par tout homme du métier.

Ainsi, on mélange la base obtenue avec un catalyseur en proportion égale, dans le cas où on désire obtenir un silicone à réaction de polyaddition.

Néanmoins, la même opération peut être effectuée mais avec des proportions de base et de catalyseur différentes dans le cas où on désire obtenir un silicone résultant d'une polycondensation.

On a par ailleurs effectué les essais suivants.

Dans un premier temps, on a vérifié le caractère de stabilité et de conformité structurelle du matériau à base de silicone obtenu selon l'invention.

On constate ainsi qu'après stockage pendant une durée de 24 mois, le matériau est parfaitement conservé.

Par ailleurs, on a observé que les temps de réticulation du silicone, c'est-à-dire de la base avec le catalyseur, se sont avérés identiques en présence ou en l'absence de l'agent antiseptique.

On a également vérifié les propriétés bactériologiques du matériau pour l'empreinte obtenue.

Ainsi, on a fabriqué trois empreintes sur des patients différents.

Chacune des empreintes a été rincée à l'eau courante dix secondes, puis mise en culture dans une cuve à 30°C et pendant trois jours.

Après 48 heures, on observe aucune culture bactérienne à la surface des empreintes.

L'invention permet donc d'obtenir un matériau à base de silicone pour empreinte dentaire qui ne présente aucun risque d'infection aussi bien dans la masse de silicone qu'à la surface de celui-ci. Il permet ainsi de remplir toutes les règles d'hygiène élémentaires vis-à-vis à la fois du dentiste, du prothésiste et de patient.

Par ailleurs, le silicone incorporant l'agent antiseptique garde sa stabilité dimensionnelle et structurelle dans le temps.

En outre et comme déjà dit, l'invention ne se limite pas au domaine des empreintes dentaires mais s'applique à tout secteur technique susceptible de mettre en oeuvre des matériaux à base de silicone qui nécessitent d'être désinfectés.

## Revendications

1. Matériau à base de silicone, notamment pour empreinte dentaire, **caractérisé en ce qu'**il comporte un agent antiseptique incorporé dans la masse du silicone et relargable progressivement jusqu'à la surface de l'empreinte, ledit agent antiseptique comprenant en mélange un agent chélatant, un agent actif sur les membranes phospholipidiques des bactéries ou virus, un agent actif sur les nucléocapsides des virus ou sur tout site récepteur des bactéries.

2. Matériau selon la revendication 1, **caractérisé en ce que** l'agent chélatant est l'acide éthylène diamine tétraacétique.

3. Matériau selon la revendication 1, **caractérisé en ce que** l'agent actif sur les membranes phospholipidiques des bactéries ou virus est un sel d'ammonium quaternaire, notamment le chlorure de benzalkonium.

4. Matériau selon la revendication 1, **caractérisé en ce que** l'agent actif sur les nucléocapsides des virus ou sur tout site récepteur des bactéries est le tosylchloramide sodique ou l'un de ses analogues chlorés.

5. Matériau selon l'une des revendications 1 à 4, **caractérisé en ce que** l'agent antiseptique comprend en masse :
- entre 8 et 80 % d'acide éthylène diamine tétraacétique ;
- entre 2 et 20 % de chlorure de benzaikonium ;
- entre 1 et 20 % de tosylchloramide sodique ou l'un de ses analogues chlorés.

6. Matériau selon l'une des revendications 1 à 5, **caractérisé en ce que** l'agent antiseptique comprend en outre un double acide aminé, notamment l'aspartam.

7. Matériau selon l'une des revendications 1 à 6, **caractérisé en ce que** l'agent antiseptique comprend en outre un agent anti-inflammatoire et cicatrisant, notamment l'allantoine

8. Matériau selon l'une des revendications 1 à 7, **caractérisé en ce que** l'agent antiseptique comprend en outre un arôme naturel et un adjuvant d'isotonocité, notamment le sorbitol.

9. Matériau selon l'une des revendications précédentes, **caractérisé en ce que** la proportion d'agent antiseptique dans ledit matériau est comprise entre 0,1 et 1 % en masse.

10. Procédé pour la fabrication d'un matériau à base de silicone pour empreinte dentaire, ledit silicone étant obtenu après réticulation d'une base organochlorosilane, ladite réticulation étant initiée par un catalyseur, **caractérisé en ce qu'**on incorpore un agent antiseptique dans la base organochlorosilane, puis **en ce qu'**on malaxe ladite base organochlorosilane avec le catalyseur afin d'obtenir après réticulation, le matériau final, ledit agent antiseptique comprenant en mélange un agent chélatant, un agent actif sur les membranes phospholipidiques des bactéries ou virus, un agent actif sur les nucléocapsides des virus ou sur tout site récepteur des bactéries

11. Procédé selon la revendication 10, **caractérisé en ce que** l'agent chélatant est l'acide éthylène diamine tétraacétique.

12. Procédé selon la revendication 10, **caractérisé que** l'agent actif sur les membranes phospholipidiques des bactéries ou virus est un sel d'ammonium quaternaire, notamment le chlorure de benzaikonium.

13. Procédé selon la revendication 10, **caractérisé en ce que** l'agent actif sur les nucléocapsides des virus ou sur tout site récepteur des bactéries est le tosylchloramide sodique ou l'un de ses analogues chlorés.

14. Procédé selon l'une des revendications 10 à 13, **caractérisé en ce que** l'agent antiseptique comprend en masse :
- de 8 à 80 % d'acide éthylène diamine tétraacétique ;
- de 2 à 20 % de chlorure de benzalkonium ;
- de 1 à 20 % de tosylchloramide sodique ou l'un de ses analogues chlorés.

15. Procédé selon l'une des revendications 10 à 14, **caractérisé en ce que** l'agent antiseptique comprend en outre un double acide aminé, notamment l'aspartam.

16. Procédé selon l'une des revendications 10 à 15, **caractérisé en ce qu'**avant incorporation de l'agent antiseptique dans la base organochlorosilane :
- on mélange préalablement l'agent antiseptique avec une huile de silicone ;
- on agite ensuite le mélange obtenu et on le laisse décanter ;
- et enfin, après filtration, on incorpore la dispersion obtenue dans la base organochlorosilane.

## Patentansprüche

1. Material auf der Basis von Silikon, insbesondere für zahnärztliche Abdrücke, **dadurch gekennzeichnet, daß** es ein antiseptisches Mittel enthält, welches in die Silikonmasse eingearbeitet ist und welches schrittweise wieder an die Oberfläche des Abdrucks abgegeben werden kann, wobei das antiseptische Mittel eine Mischung aus einem Chelatbildner, einem auf die Phospholipidmembranen der Bakterien oder Viren einwirkenden Mittel, einem auf die Nukleocapside der Viren oder auf alle Rezeptorstellen der Bakterien einwirkenden Mittel umfaßt.

2. Material nach Anspruch 1, **dadurch gekennzeichnet, daß** der Chelatbildner Ethylendiamintetraessigsäure ist.

3. Material nach Anspruch 1, **dadurch gekennzeichnet, daß** das auf die Phospholipidmembranen der Bakterien oder Viren einwirkende Mittel ein quartäres Ammoniumsalz, insbesondere Benzalkoniumchlorid ist.

4. Material nach Anspruch 1, **dadurch gekennzeichnet, daß** das auf die Nukleocapside der Viren oder auf alle Rezeptorstellen der Bakterien einwirkende Mittel Tosylchloramid-Natrium oder eins von dessen chlorierten Analogen ist.

5. Material nach einem der Ansprüche 1 bis 4, **dadurch gekennzeichnet, daß** das antiseptische Mittel in Bezug auf die Masse umfaßt:
- zwischen 8 und 80 % Ethylendiamintetraessigsäure;
- zwischen 2 und 20 % Benzalkoniumchlorid;
- zwischen 1 und 20 % Tosylchloramid-Natrium oder eins von dessen chlorierten Analogen.

6. Material nach einem der Ansprüche 1 bis 5, **dadurch gekennzeichnet, daß** das antiseptische Mittel unter anderem eine zweifache Aminosäure, insbesondere Aspartam umfaßt.

7. Material nach einem der Ansprüche 1 bis 6, **dadurch gekennzeichnet, daß** das antiseptische Mittel unter anderem ein entzündungshemmendes und die Wundheilung förderndes Mittel, insbesondere Allantoin umfaßt.

8. Material nach einem der Ansprüche 1 bis 7, **dadurch gekennzeichnet, daß** das antiseptische Mittel unter anderem ein natürliches Aroma und einen Isotoniezusatz, insbesondere Sorbitol umfaßt.

9. Material nach einem der vorhergehenden Ansprüche, **dadurch gekennzeichnet, daß** der Anteil des antiseptischen Mittels in dem genannten Material zwischen 0,1 und 1 Massen-% liegt.

10. Verfahren zur Herstellung eines Materials auf der Basis von Silikon für zahnärztliche Abdrücke, wobei das genannte Silikon nach Vernetzung eines Organochlorsilangrundstoffes erhalten wurde, wobei die genannte Vernetzung durch einen Katalysator gestartet wurde, **dadurch gekennzeichnet, daß** man ein antiseptisches Mittel in den Organochlorsilangrundstoff einarbeitet, daß man dann den genannten Organochlorsilangrundstoff mit dem Katalysator verknetet, um nach Vernetzung das endgültige Material zu erhalten, wobei das genannte antiseptische Mittel eine Mischung aus einem Chelatbildner, einem auf die Phospholipidmembranen der Bakterien oder Viren einwirkenden Mittel, einem auf die Nukleocapside der Viren oder auf alle Rezeptorstellen der Bakterien einwirkenden Mittel umfaßt.

11. Verfahren nach Anspruch 10, **dadurch gekennzeichnet, daß** der Chelatbildner Ethylendiamintetraessigsäure ist.

12. Verfahren nach Anspruch 10, **dadurch gekennzeichnet, daß** das auf Phospholipidmembranen der Bakterien oder Viren einwirkende Mittel ein quartäres Ammoniumsalz, insbesondere Benzalkoniumchlorid ist.

13. Verfahren nach Anspruch 10, **dadurch gekennzeichnet, daß** das auf die Nukleocapside der Viren oder auf alle Rezeptorstellen der Bakterien einwirkende Mittel Tosylchloramid-Natrium oder eins von dessen chlorierten Analogen ist.

14. Verfahren nach einem der Ansprüche 10 bis 13, **dadurch gekennzeichnet, daß** das antiseptische Mittel in Bezug auf die Masse umfaßt:
- von 8 und 80 % Ethylendiamintetraessigsäure;
- von 2 und 20 % Benzalkoniumchlorid;
- von 1 und 20 % Tosylchloramid-Natrium oder eins von dessen chlorierten Analogen.

15. Verfahren nach einem der Ansprüche 10 bis 14, **dadurch gekennzeichnet, daß** das antiseptische Mittel unter anderem eine zweifache Aminosäure, insbesondere Aspartam umfaßt.

16. Verfahren nach einem der Ansprüche 10 bis 15, **dadurch gekennzeichnet, daß** man vor dem Einarbeiten des antiseptischen Mittels in den Organochlorsilangrundstoff:
- vorher das antiseptische Mittel mit einem Silikonöl mischt;
- anschließend die erhaltene Mischung schüttelt und diese sich absetzen läßt;
- und schließlich, nach Filtration, die erhaltene Dispersion in den Organochlorsilangrundstoff einarbeitet.

## Claims

1. A silicone-based material, in particular for dental impression, **characterized in that** it comprises an antiseptic agent incorporated into the mass of the silicone and which can be gradually released up to the surface of the impression, said antiseptic agent comprising, in the form of a mixture, a chelating agent, an agent which is active on the phospholipid membranes of bacteria or viruses, an agent which is active on the nucleocapsids of viruses or on any bacteria receptor site.

2. The material as claimed in claim 1, wherein the chelating agent is ethylenediaminetetraacetic acid.

3. The material as claimed in claim 1, wherein the agent which is active on the phospholipid membranes of bacteria or viruses is a quaternary ammonium salt, in particular benzalkonium chloride.

4. The material as claimed in claim 1, wherein the agent which is active on the nucleocapsids of viruses or on any bacteria receptor site is sodium tosylchloramide or one of its chlorinated analogs.

5. The material as claimed in one of claims 1 to 4, wherein the antiseptic agent comprises by mass:
- between 8 and 80% of ethylenediaminetetraacetic acid;
- between 2 and 20% of benzalkonium chloride;
- between 1 and 20% of sodium tosylchloramide or one of its chlorinated analogs.

6. The material as claimed in one of claims 1 to 5, wherein the antiseptic agent comprises, in addition, a double amino acid, in particular aspartame.

7. The material as claimed in one of claims 1 to 6, wherein the antiseptic agent comprises, in addition, an antiinflammatory and cicatrizing agent, in particular allantoin.

8. The material as claimed in one of claims 1 to 7, wherein the antiseptic agent comprises, in addition, a natural flavoring and an isotonicity adjuvant, in particular sorbitol.

9. The material as claimed in the preceding claims, wherein the proportion of antiseptic agent in said material is between 0.1 and 1% by mass.

10. A method of producing a silicone-based dental impression material, said silicone being obtained after the crosslinking of an organochlorosilane base, said crosslinking being initiated by a catalyst, wherein an antiseptic agent is incorporated into the organo-chlorosilane base and then said organo-chlorosilane base is mixed with the catalyst in order to obtain the final material after crosslinking, said antiseptic agent comprising in the form of a mixture a chelating agent, an agent which is active on the phospholipid membranes of bacteria or viruses, and an agent which is active on the nucleocapsids of viruses or on any bacteria receptor site.

11. The method as claimed in claim 10, wherein the chelating agent is ethylenediaminetetraacetic acid.

12. The method as claimed in claim 10, wherein the agent which is active on the phospholipid membranes of bacteria or viruses is a quaternary ammonium salt, in particular benzalkonium chloride.

13. The method as claimed in claim 10, wherein the agent which is active on the nucleocapsids of viruses or on any bacteria receptor site is sodium tosylchloramide or one of its chlorinated analogs.

14. The method as claimed in one of claims 10 to 13, wherein the antiseptic agent comprises by mass:
- from 8 to 80% of diethylenediaminetetraacetic acid;
- from 2 to 20% of benzalkonium chloride;
- from 1 to 20% of sodium tosylchloramide or one of its chlorinated analogs.

15. The method as claimed in one of claims 10 to 14, wherein the antiseptic agent comprises, in addition, a double amino acid, in particular aspartame.

16. The method as claimed in one of claims 10 to 15, wherein before incorporating the antiseptic agent into the organochlorosilane base:
- the antiseptic agent is mixed beforehand with a silicone oil,
- the mixture obtained is then stirred and it is allowed to separate by settling,
- and finally, after filtration, the dispersion obtained is incorporated into the organo-chlorosilane base.
